(19) Europäisches Patentamt
European Patent Office
Office européen des brevets

(11) **EP 4 431 932 A1**

(12) **EUROPEAN PATENT APPLICATION**

(43) Date of publication:
**18.09.2024 Bulletin 2024/38**

(51) International Patent Classification (IPC):
**G01N 27/624** (2021.01)    **G01N 33/00** (2006.01)
**G01N 1/22** (2006.01)    **G01N 1/44** (2006.01)

(21) Application number: **24158479.6**

(22) Date of filing: **19.02.2024**

(52) Cooperative Patent Classification (CPC):
**G01N 33/0047; G01N 27/624; G01N 27/66;
G01N 33/006;** G01N 33/0065; G01N 33/0068;
G01N 2035/00455

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.03.2023 CN 202310235413**

(71) Applicant: **Honeywell International Inc.
Charlotte, NC 28202 (US)**

(72) Inventors:
• **LIANG, Feng**
**Charlotte, 28202 (US)**
• **YANG, Fan**
**Charlotte, 28202 (US)**
• **HU, Yu**
**Charlotte, 28202 (US)**
• **CHEN, Guodong**
**Charlotte, 28202 (US)**
• **TIAN, Renbing**
**Charlotte, 28202 (US)**
• **ZHU, Hao**
**Charlotte, 28202 (US)**

(74) Representative: **Haseltine Lake Kempner LLP
Cheapside House
138 Cheapside
London EC2V 6BJ (GB)**

(54) **PHOTO IONIZATION DETECTOR AND METHODS OF USING THE SAME**

(57) A method (700) for measuring a volatile organic compound (VOC) by a controller component (101) of a photo ionization detector (PID) (100; 600) is provided. The method (700) includes: measuring a relative humidity (201, 201') and a temperature of a gas that is input into the PID (100; 600); comparing the measured relative humidity (201, 201') with a predetermined humidity threshold; in an instance in which the relative humidity (201') is higher than the predetermined humidity threshold: determining a drift signal of a signal electrode (105a) of the PID (100; 600) when an ionization device (102) of the PID (100; 600) is off between different cycles; comparing the drift signal with a predetermined drift threshold; in an instance in which the drift signal is greater than the predetermined drift threshold, switching on a heating component (107) of the PID (100; 600) to reduce the relative humidity (201'); and in an instance in which the drift signal is smaller than the predetermined drift threshold, switching off the heating component (107); and measuring a concentration of the VOC of the gas.

FIG. 1

EP 4 431 932 A1

**Description**

**FIELD OF THE INVENTION**

Exemplary embodiments of the present disclosure relate generally to a photo ionization detector, and more particularly, in some examples, to a photo ionization detector with a heating component working in a pulse mode.

**BACKGROUND**

Photo ionization detector (PID) may be used to monitor air quality and/or industrial pollution by detecting the presence and/or measure the concentration of a volatile organic compound (VOC) in a gas, such as ambient air. However, detecting the concentration of the VOC in gasses through many traditional VOC detectors can be affected by relative humidity. Through applied effort, ingenuity, and innovation, many of these identified problems have been solved by developing solutions that are included in embodiments of the present disclosure, many examples of which are described in detail herein.

**BRIEF SUMMARY**

Various embodiments described herein relate to methods, apparatuses, and systems for improving sensitivity and performance of an apparatus such as, for example, a flow sensing apparatus.

In accordance with various examples of the present disclosure, a method for measuring a volatile organic compound (VOC) by a controller component of a photo ionization detector (PID) is provided. The method includes measuring a relative humidity and a temperature of a gas that is input into the PID; comparing the measured relative humidity with a predetermined humidity threshold; in an instance in which the relative humidity is higher than the predetermined humidity threshold: determining a drift signal when an ionization device is off between different cycles; comparing the drift signal with a predetermined drift threshold; in an instance in which the drift signal is greater than the predetermined drift threshold, switching on a heating component to reduce the relative humidity; and in an instance in which the drift signal is smaller than the predetermined drift threshold, switching off the heating component; and measuring a concentration of the VOC of the gas.

In some embodiments, the method further comprises turning the ionization device on and off periodically to create a plurality of ionized gas molecules by exposing the gas to the ionization device, where the plurality of ionized gas molecules are detected by signal electrodes, and the signal electrodes are electrically coupled to the controller component of the PID.

In some embodiments, the controller component is configured to provide a direct current (DC) potential to the signal electrodes, such that the VOC is separated and detected.

In some embodiments, the predetermined humidity threshold is determined based on a temperature of the gas.

In some embodiments, measuring the concentration of the VOC in the gas comprises: determining a first raw signal after the ionization device is on for a first predetermined time; determining a second raw signal after the ionization device is off for a second predetermined time; and calculating the concentration of the VOC according to the first raw signal and the second raw signal.

In some embodiments, determining the drift signal when the ionization device is off between different cycles comprises: determining a third raw signal after the ionization device is off for a third predetermined time in a first cycle; determining a fourth raw signal after the ionization device is off for the third predetermined time in a second cycle; and calculating the concentration of the VOC according to the third raw signal and the fourth raw signal.

In some embodiments, the heating component is located in a detecting region of the gas.

In some embodiments, the ionization device is an ultraviolet lamp projecting into the detecting region of the gas, and gas molecules with an ionization potential lower than ionization energy of the UV light are ionized.

In some embodiments, a duty cycle of the ultraviolet lamp is in a range from 10% to 70%.

In some embodiments, the relative humidity and temperature of the gas are measured by a humidity and temperature sensor located in the detecting region of the gas.

In some example embodiments, a photo ionization detector (PID) for measuring a volatile organic compound (VOC) is provided. The PID includes a controller component; signal electrodes; a heating component; an ionization device, wherein the controller component is configured to: measure a relative humidity and a temperature of a gas that is input into the PID; compare the measured relative humidity with a predetermined humidity threshold; in an instance in which the relative humidity is higher than the predetermined humidity threshold, determine a drift signal of an ionization device; compare the drift signal with a predetermined drift threshold; in an instance in which the drift signal is greater than the predetermined drift threshold, switch on a heating component to reduce the relative humidity; and in an instance in which the drift signal is smaller than the predetermined drift threshold, switch off the heating component.

In some embodiments, the controller component is further configured to turn the ionization device on and off periodically to create a plurality of ionized gas molecules by exposing the gas to the ionization device, and the plurality of ionized gas molecules are detected by signal electrodes, and the signal electrodes are electrically coupled to the controller component.

In some embodiments, the controller component is further configured to provide a direct current (DC) potential to the signal electrodes, such that the VOC is separated and detected.

In some embodiments, the predetermined humidity threshold is determined based on a temperature of the gas.

In some embodiments, to measure the concentration of the VOC in the gas, the controller component is configure to: determine a first raw signal after the ionization device is on for a first predetermined time; determine a second raw signal after the ionization device is off for a second predetermined time; and calculate the concentration of the VOC according to the first raw signal and the second raw signal.

In some embodiments, to determine the drift signal when the ionization device is off between different cycles, the controller component is configure to: determine a third raw signal after the ionization device is off for a third predetermined time in a first cycle; determine a fourth raw signal after the ionization device is off for the third predetermined time in a second cycle; and calculate the concentration of the VOC according to the third raw signal and the fourth raw signal.

In some embodiments, the heating component is located in a detecting region of the gas.

In some embodiments, the ionization device is an ultraviolet lamp projecting into the detecting region of the gas, and gas molecules with an ionization potential lower than ionization energy of the UV light are ionized.

In some embodiments, a duty cycle of the ultraviolet lamp is in a range from 10% to 70%.

In some embodiments, the relative humidity and temperature of the gas are measured by a humidity and temperature sensor located in the detecting region of the gas.

## BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:

FIG. 1 illustrates a schematic diagram depicting a photo ionization detector (PID) for measuring a volatile organic compound (VOC) in a gas, in accordance with various embodiments of the present disclosure;

FIG. 2 illustrates a schematic diagram depicting a signal sequence of a photo ionization detector (PID) for measuring a volatile organic compound (VOC) in a gas, in accordance with various embodiments of the present disclosure;

FIG. 3 illustrates a schematic diagram depicting a signal sequence of an ionization device, in accordance with various embodiments of the present disclosure;

FIG. 4 illustrates a schematic diagram depicting a signal sequence 400 of a photo ionization detector (PID) for measuring a volatile organic compound (VOC) in a gas, in accordance with various embodiments of the present disclosure;

FIG. 5 illustrates a schematic diagram depicting signal intensities of raw signals of a PID, in accordance with various embodiments of the present disclosure is provided;

FIG. 6 illustrates a schematic block diagram depicting an example photo ionization detector (PID) for measuring a volatile organic compound (VOC) in a gas, in accordance with various embodiments of the present disclosure is provided;

FIG. 7 illustrates a flowchart diagram illustrating an example method for measuring a volatile organic compound (VOC) in a gas, in accordance with various embodiments of the present disclosure is provided;

FIG. 8 illustrates a flowchart diagram illustrating an example method for measuring a concentration of the VOC of the gas, in accordance with various embodiments of the present disclosure is provided; and

FIG. 9 illustrates a flowchart diagram illustrating an example method for determining a drift signal when the ionization device is off between different cycles, in accordance with various embodiments of the present disclosure is provided.

## DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in a dashed line for visibility of the underlying components.

The phrases "in an example embodiment," "some embodiments," "various embodiments," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such components or features may be optionally included in some embodiments, or may be excluded.

The term "electronically coupled" or "in electronic communication with" in the present disclosure refers to two or more electrical elements (for example, but not limited to, an example processing circuitry, communication module, input/output module memory, humidity sensing component, cooling element, gas detection component) and/or electric circuit(s) being connected through wired means (for example but not limited to, conductive wires or traces) and/or wireless means (for example but not limited to, wireless network, electromagnetic field), such that data and/or information (for example, electronic indications, signals) may be transmitted to and/or received from the electrical elements and/or electric circuit(s) that are electronically coupled.

Various example embodiments address technical problems associated with detecting a volatile organic compound (VOC) in a gas. As understood by those of skill in the field to which the present disclosure pertains and in some examples, there are numerous example scenarios in which a user may need to detect the presence and/or measure the concentration of VOC in a gas (e.g., air).

Volatile organic compounds (VOCs) are substances associated with a low boiling point at room temperature resulting in molecules that are susceptible to ready release into the surrounding air. VOCs released into the air can be dangerous to human health or to the environment. For example, substances such as solvents and paint thinners, as well as vapors associated with fuel and oil, may release VOCs into the surrounding environment creating polluted air, smog and/or generating ozone. VOCs inhaled by humans and animals can have adverse health effects such as causing irritation, allergies, damage to organs and the nervous system, and even cancer. In addition, and in some examples, a detectable amount of VOCs may be released from drugs, explosives, and chemical weapons, indicating the presence of these objects in a particular environment.

In some examples, a photo ionization detector (PID) may be used to monitor air quality and/or industrial pollution by detecting the presence and/or measure concentrations of VOCs through a technique known as ion mobility spectrometry (IMS). IMS exploits the differing mobility of ionized molecules in a gas, including VOCs, to separate a targeted VOC from other ionized and non-ionized molecules. Once separated, a device utilizing IMS techniques may measure the presence of ionized modules based on the voltage produced on an electrical conductor. High-field asymmetric-waveform ion-mobility spectrometry (FAIMS), is another technique that takes advantage of the differing ion mobilities to separate ionized gas molecules. Specifically, FAIMS leverages the property present in many ionized molecules that the mobility of the ionized molecule is different in the presence of a high electric field versus a low electric field. PID utilizing FAIMS techniques may pass the gas potentially containing VOCs through a varying electric field. Ionized molecules that are not targeted will drift toward one or the other of the electrodes where they will be neutralized upon contact. While targeted molecules, in some examples, will move through the separation region without impacting either electrode. Molecules that pass through the separation region without being neutralized will be detected, in some examples, and the number of remaining molecules will be calculated based on the electrical voltage produced by their presence. Detecting the presence and/or measuring the concentration of VOCs in gasses can be an important tool, in some examples, in identifying airborne molecules that are harmful to humans and/or the environment, or even indicate the presence of dangerous and/or illegal items.

However, detecting a concentration of a VOC in a gas by many traditional VOC detectors can be affected, in some examples, by the relative humidity of the detecting region. Traditional PID is capable of measurements from 0 to 10,000 ppm with resolution as low as 1 parts per million (PPM). To improve the accuracy of detecting the concentration of the VOC in a gas, many VOC detectors require a dehumidifier to reduce the relative humidity of the detecting region, adding to the cost, size and complexity of the device. Finally, PID generally requires an ionization source which is constantly enabled to ionize the gas, decreasing the project life of the device.

The various example embodiments described herein utilize various techniques to improve the accuracy of detection of the VOC in a gas using FAIMS techniques. For example, in some embodiments, a PID in accordance with the present disclosure may utilize heating component to keep the relative humidity of the detecting region stable. In embodiments where the heating component is utilized to keep the relative humidity low, the overall accuracy of the sensor may be improved. For example, the PID in the present disclosure is capable of measurements from 0 to 10,000 ppm with resolution as low as 1 parts per billion (PPB).

In some examples, a drift of concentration of the VOC between different cycles may be determined when the ionization device is off. In some examples, the heating component may be turned on in response to the relative humidity is higher than a predetermined humidity threshold and the drift of concentration of the VOC is greater than a predetermined drift threshold

In addition, in some embodiments disclosed herein, turning an ionization device on and off periodically allows the ionization device to last longer, in some examples. In embodiments where the ionization device is an ultra-violet (UV) lamp, or similar device, the UV light from the UV lamp has high energies and the material of the PID may decay and may be damaged because of exposure to the UV light.

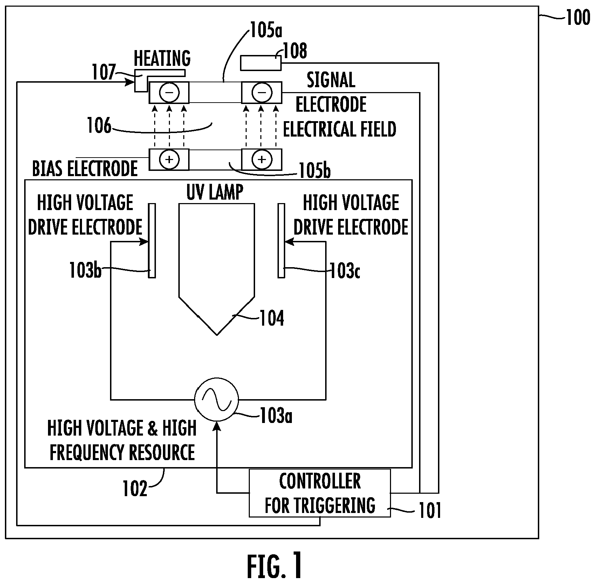

Referring now to FIG. 1, a schematic diagram depicting a photo ionization detector (PID) for measuring a volatile organic compound (VOC) in a gas, in accordance with various embodiments of the present disclosure is provided. As depicted in FIG. 1, a PID 100 for measuring a volatile organic compound (VOC) in a gas includes a controller component 101, an ionization device 102, signal electrodes 105 (105a and 105b), a heating component 107, and a humidity and temperature sensor 108.

As illustrated in FIG. 1, the controller component 101 may be electrically connected to the ionization device 102, the signal electrodes 105 (105a and 105b), the heating component 107, and the humidity and temperature sensor 108. In some examples, the controller component 101 is a circuit board with micro-controller to communicate with the ionization device 102, the signal electrodes 105, and the heating component 107. For example, the controller component 101 may receive signals from the signal electrodes 105. For example, the controller component 101 may turn on and off the ionization device 102 periodically, such that the PID is able to work in a pulse mode. For example, the controller component 101 may turn on or turn off the heating component 107. For example, the controller component 101 may communicate with the humidity and temperature sensor 108 to measure and/or monitor the relative humidity and the temperature of the gas in a detecting region 106 of the PID 100 .

In various embodiments, the humidity and temperature sensor 108 may measure and/or monitor a relative humidity and a temperature of the gas. In various embodiments, the heating component 107 is located adjacent to the signal electrodes 105 and has no direct contact with the signal electrodes 105.

In various embodiments, the ionization device 102 includes a power source 103a, driving electrodes 103b and 103c electrically connected with the power source 103a, and a UV lamp 104 located between the driving electrodes 103b and 103c.

In some embodiments, the UV lamp 104 may be directed at the detecting region 106 of the PID 100. For example, the UV lamp 104 may emit photons of different energies based on an inert gas within the photon lamp. In some examples, photons of different energies may be emitted by the UV lamp 104 and enter the detecting region of the PID 100. The photons of different energies function to ionize a gas that is input into the detecting region 106 of the PID 100 and create a plurality of ionized gas molecules, thus ionizing a different range of potentially detectable VOC. For example, the inert gas of the UV lamp 104 may contain Krypton, Xenon, Argon, etc.

In some embodiments, the driving electrodes 103b and 103c may be high voltage drive electrodes. For example, the controller component 101 may be electrically connected to the power source 103a and be configured to instruct the power source 103a to apply an alternating current (AC) voltage between the driving electrodes 103b and 103c during the operation of the PID 100. For example, the AC voltage may be a high voltage and high frequency signal. In some embodiments, the AC voltage may turn on and off the ionization device 102 periodically to create a plurality of ionized gas molecules by exposing the gas to the UV light. For example, the ionization device 102 is on or active when the AC voltage is high and the ionization device 102 is off or deactivated when the AC voltage is low.

In some examples, turning on and off the ionization device 102 may prolong the life of the ionization device 102, particularly, in an instance in which the ionization device 102 includes a UV lamp. For example, instead of being ON or activated all the working time, the ionization device 102 is turned ON and OFF at every measurement to achieve a longer service life, in some examples. For example, the PID working in a continuous short pulse mode of the present disclosure are capable of extending the service life of the PID up to 18,000 hours, in some examples, as compared to 5,000 hours of the traditional PID. In some examples, the UV lamp of the PID may be working in a pulse mode with a pulse duration in a range of 10 ms-100 ms. In some examples, a duty cycle of the pulse may be in a range of 1%-80%. For example, a pulse duration of the short pulse may be 50 ms and a pulse length of the short pulse may be 2000 ms.

In some embodiments, as illustrated in FIG. 1, the detecting region 106 may be a detecting chamber defined between the signal electrode 105a and the bias electrode 105b for the photo ionization process and the signal collection process.

In various embodiments, the VOC of the gas that is input into the detecting region 106 of the PID 100 may be ionized by the UV light from the UV lamp 104 and ionized gas molecules may be created in the detecting region 106 of the PID 100. The ionized gas molecules may carry an electric charge after the VOC is ionized. As such, the ionized gas molecules may become responsive to an electric field formed between the signal electrode 105a and the bias electrode 105b. For example, the controller component 101 may be configured to apply a direct current (DC) voltage between the signal electrode 105a and the bias electrode 105b to create an electric field between the signal electrode 105a and the bias electrode 105b. For example, the ionized gas molecules may be subjected to the electric field between the signal electrode 105a and the bias electrode 105b may be forced to move towards the signal electrode 105a. In some examples, ionized gas molecules may be de-ionized when they contact the signal electrode 105a and a corresponding current may be generated. As such, ionized gas molecules may be detected when the corresponding current from the signal electrode 105a is output to the controller component 101. In some examples, an amplitude of the corresponding current is a function of the number of the ionized gas molecules. For example, the amplitude of the corresponding current may be proportional to the number of the ionized gas molecules. In some examples, the controller component 101 may detect the VOC and/or determine a concentration of the VOC according to the amplitude of the corresponding current.

In various embodiments, the heating component 107 may take the form of an electric heater and may be located adjacent to the signal electrode 105a. For example, the controller component 101 may be electrically connected to heating component 107 and be configured to turn on the heating component 107 to heat the gas in the detecting region 106, such that a relative humidity of the gas in the detecting region 106 may decrease. In an instance in which the relative humidity of the gas in the detecting region 106 becomes lower than a predetermined humidity threshold, the controller component 101 may be configured to turn off the heating component 107.

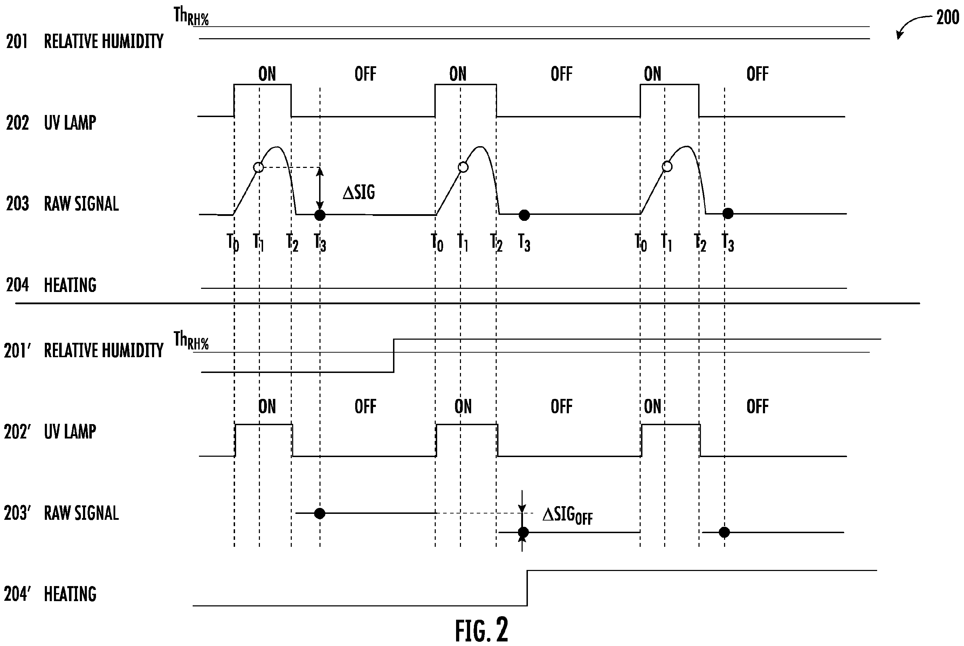

Referring now to FIG. 2, a schematic diagram depicting a signal sequence 200 of a photo ionization detector (PID) for measuring a volatile organic compound (VOC) in a gas, in accordance with various embodiments of the present disclosure is provided.

As shown in a top portion of FIG. 2 includes one or more of a relative humidity 201 of the detecting region 106, an output signal 202 of the power source 103a, a raw signal 203 of the signal electrode 105a, and a status signal 204 of the heating component 107 are illustrated in an instance in which the relative humidity 201 of the detecting region 106 is lower than a predetermined humidity threshold $TH_{RH}\%$. In some examples, the predetermined humidity threshold $TH_{RH}\%$ may be determined based on the temperature. In some embodiments, the predetermined humidity threshold $TH_{RH}\%$ may be in a range from 30% to 100%. In yet further embodiments, the predetermined humidity threshold $TH_{RH}\%$ may be 90%.

In various embodiments, the relative humidity 201 of the detecting region 106 is measured and/or monitored by the humidity and temperature sensor 108, and the power source 103a provides the output signal 202 to the UV lamp 104. When the output signal 202 is low, the UV lamp is off, and when the output signal 202 is high, the UV lamp is on. As shown in the top portion of FIG. 2, the UV lamp is configured to be turned on and turned off periodically when the PID 100 is enabled to detect and/or measure the VOC. When the PID 100 is operating, a raw signal 203 of the signal electrode 105a is communicated to the controller component 101.

As further shown in the top portion of FIG. 2, in an example first cycle, the UV lamp starts to turn on at time $T_0$. The raw signal 203 is recorded as a first raw signal $Sig_{on}$ at time $T_1$. The UV lamp starts to turn off at time $T_2$. The raw signal 203 is recorded as a second raw signal $Sig_{off}$ at time $T_3$. In some examples, the controller component 101 may determine a concentration of the VOC in the gas based on a valid signal $\Delta Sig$, where $\Delta Sig = Sig_{on} - Sig_{off}$. For example, the VOC may be proportional to the valid signal $\Delta Sig$.

As shown in a bottom portion of FIG. 2, a relative humidity 201' of the detecting region 106, an output signal 202' of the power source 103a, a raw signal 203' of the signal electrode 105a, and a status signal 204' of the heating component 107 are illustrated in an instance in which the relative humidity 201 of the detecting region 106 may change to be higher than a predetermined humidity threshold $TH_{RH}\%$.

In various embodiments, the relative humidity 201' of the detecting region 106 is monitored by the humidity and temperature sensor 108, and the power source 103a provides the output signal 202' to the UV lamp 104. When the output signal 202' is low, the UV lamp is off, and when the output signal 202' is high, the UV lamp is on. As shown in the bottom portion of FIG. 2, the UV lamp is configured to be turned on and turned off periodically when the PID 100 is enabled to detect and/or measure the VOC. When the PID 100 is operating, a raw signal 203' of the signal electrode 105a is communicated to the controller component 101.

In various embodiments, when the relative humidity 201' of the detecting region 106 changes to be higher than the predetermined humidity threshold $TH_{RH}\%$, the effects of the higher humidity on the raw signal 203' may be measured.

As further shown in the bottom portion of FIG. 2, in an example first cycle during which the relative humidity 201' of the detecting region 106 is lower than the predetermined humidity threshold $TH_{RH}\%$, the UV lamp starts to turn off at time $T_2$. The raw signal 203 is recorded as a third raw signal $Sig_{off1}$ at time $T_3$ of the example first cycle. In an example second cycle during which the relative humidity 201' of the detecting region 106 is higher than the predetermined humidity threshold $TH_{RH}\%$, the raw signal 203 is recorded as a fourth raw signal $Sig_{off2}$ at time $T_3$ of the example second cycle.

In some examples, the controller component 101 may determine whether the heating component 107 needs to be turned on based on a drift signal $\Delta Sig_{off}$ and a predetermined drift threshold $TH_{\Delta Sigoff}$, where $\Delta Sig_{off} = Sig_{off1} - Sig_{off2}$. For example, the controller component 101 may determine to turn on the heating component 107 in an instance in which the drift signal $\Delta Sig_{off}$ is greater than the predetermined drift threshold $TH_{\Delta Sigoff}$, For example, the controller component 101 may determine not to turn on the heating component 107 in an instance in which the drift signal $\Delta Sig_{off}$ is smaller than the predetermined drift threshold $TH_{\Delta Sigoff}$.

In some examples, the predetermined drift threshold $TH_{\Delta Sigoff}$ may be set according to different heating levels. For example, when the heating level is low (the duty cycle of the UV lamp is around 20%), the predetermined drift threshold $TH_{\Delta Sigoff}$ may have a small deviation. For example, when the heating level is mild (the duty cycle of the UV lamp is around 40%), the predetermined drift threshold $TH_{\Delta Sigoff}$ may have a medium deviation. For example, when the heating level is high (the duty cycle of the UV lamp is around 60%), the predetermined drift threshold $TH_{\Delta Sigoff}$ may have a large deviation. In some examples, the predetermined drift threshold $TH_{\Delta Sigoff}$ may also depends on the system architecture of the PID.

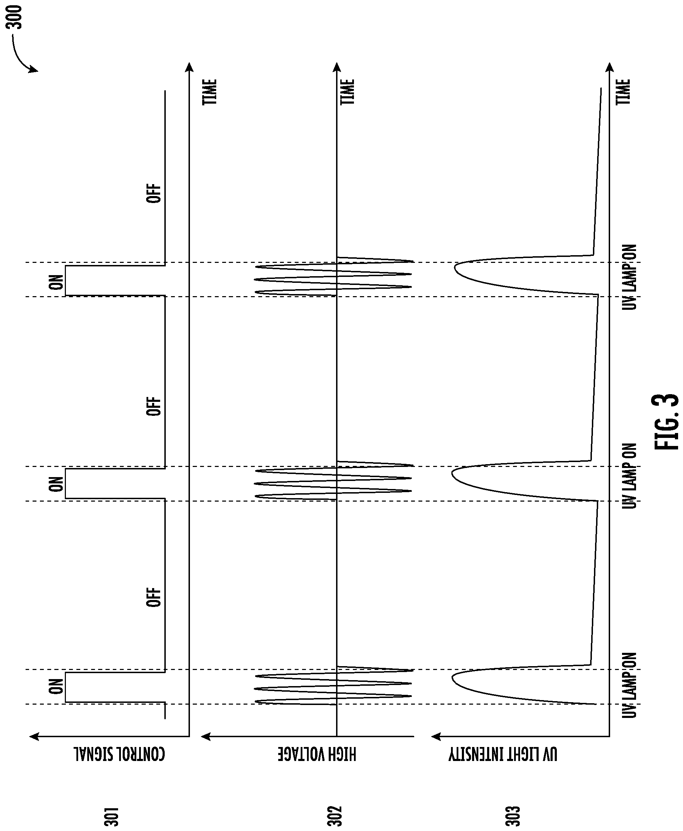

Referring now to FIG. 3, a schematic diagram depicting a signal sequence 300 of an ionization device, in accordance with various embodiments of the present disclosure is provided.

As shown in FIG. 3, the signal sequence 300 includes a control signal 301, an AC voltage signal 302, and a UV light intensity signal 303. In some embodiments, the controller component 101 is configured to provide a control signal to the power source 103a of the ionization device 102. The control signal will be high and low periodically, such that the ionization device 102 may be turned on and off, respectfully. When the control signal is on, the power source 103a may be configured to apply an AC voltage between the driving electrodes 103b and 103c.

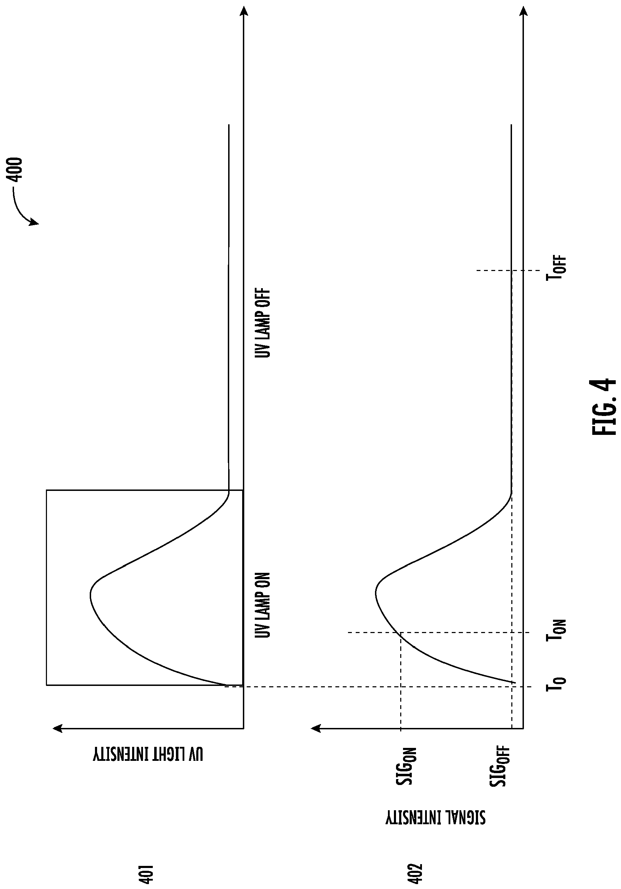

Referring now to FIG. 4, a schematic diagram depicting a signal sequence 400 of a PID 100, in accordance with various embodiments of the present disclosure is provided.

As shown in FIG. 4, the signal sequence 400 includes a UV light intensity signal 401, and an intensity signal of the raw signal 402. In some embodiments and in an example cycle, the UV lamp starts to turn on at time $T_0$. The intensity signal of the raw signal 402 is recorded as a first raw signal $Sig_{on}$ at time $T_{on}$. The intensity signal of the raw signal 402 is recorded as a second raw signal $Sig_{off}$ at time $T_{off}$ after the UV lamp is turned off for a predetermined time. In some examples, the controller component 101 may determine a concentration of the VOC in the gas based on a valid signal $\Delta Sig$, where $\Delta Sig = Sig_{on} - Sig_{off}$.

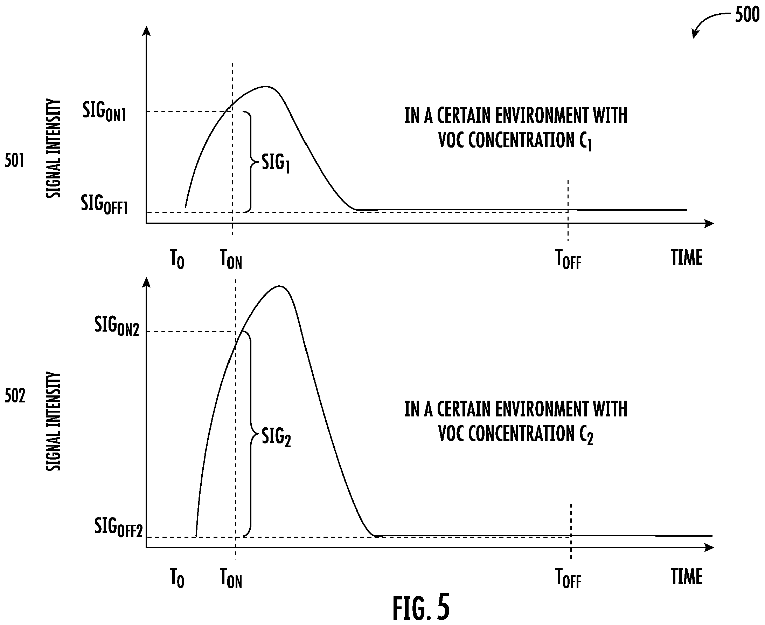

Referring now to FIG. 5, a schematic diagram depicting signal intensities of raw signals 500 of a PID 100, in accordance with various embodiments of the present disclosure is provided.

In various embodiments, the second raw signal $Sig_{off}$ may be affected by environment factors, such as the relative humidity and the temperature. In some examples, the raw signal of the signal electrode 105a may be calibrated to reduce the background noise due to the variations of the relative humidity and the temperature. As shown in FIG. 5, starting from the top, a first signal intensity 501 is measured by the PID 100 when the PID 100 is placed in a gas with a known VOC concentration $C_1$. The controller component 101 may determine a first valid signal $\Delta Sig_1$, where $\Delta Sig_1 = Sig_{on1} - Sig_{off1}$ At the bottom, a second signal intensity 502 is measured by the PID 100 when the PID 100 is placed in a gas with a known VOC concentration $C_2$. The controller component 101 may determine a second valid signal $\Delta Sig_2$, where $\Delta Sig_2 = Sig_{on2} - Sig_{off2}$.

In some embodiments, a trendline may be used to calculate a real signal $\Delta Sig$ of the signal electrode 105a. In some examples, the trendline may be a linear trendline, a logarithmic trendline, or a polynomial trendline. For example, the real signal $\Delta Sig$ may be determined according to following equation (Eq. 1)

$$\Delta Sig = \frac{C_1 - C_2}{sig_1 - sig_2} Sig_{valid} + \frac{C_2 Sig_1 - C_1 Sig_2}{sig_1 - sig_2} \qquad \text{Eq. 1}$$

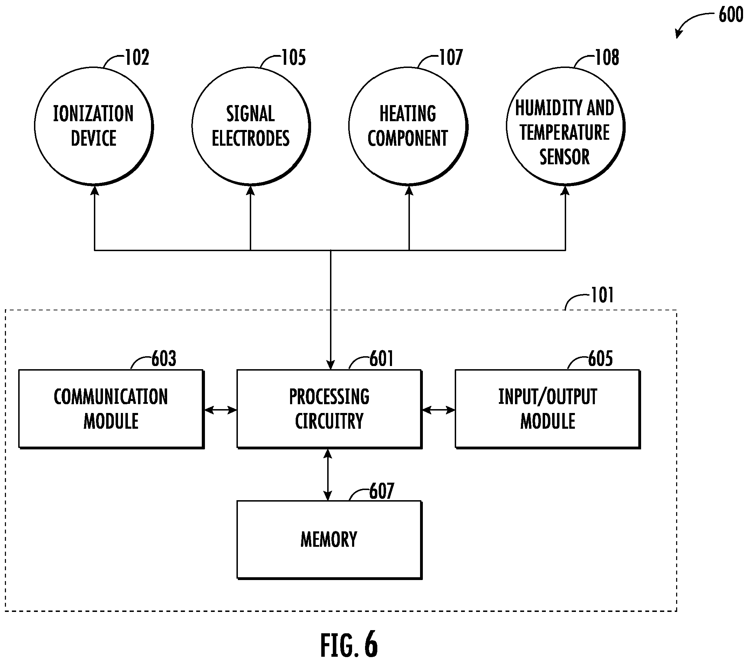

Referring now to FIG. 6, a schematic block diagram depicting an example photo ionization detector (PID) 600 for measuring a volatile organic compound (VOC) in a gas in accordance with various embodiments of the present disclosure is provided. As shown, the photo ionization detector (PID) 600 includes a controller component 101, an ionization device 102, signal electrodes 105 (105a and 105b), a heating component 107, and a humidity and temperature sensor 108, where the ionization device 102, the signal electrodes 105 (105a and 105b), the heating component 107, and the humidity and temperature sensor 108 are in electronic communication with the controller component 101. The controller component 101 includes processing circuitry 601, a communication module 603, an input/output module 605, a memory 607 and/or other components configured to perform various operations, procedures, functions or the like described herein.

As shown, the controller component 101 (such as the processing circuitry 601, communication module 603, input/output module 605 and memory 607) is electrically coupled to and/or in electronic communication with the ionization device 102, the signal electrodes 105 (105a and 105b), the heating component 107, and the humidity and temperature sensor 108. As depicted, the ionization device 102, the signal electrodes 105 (105a and 105b), the heating component 107, and the humidity and temperature sensor 108 may exchange (e.g., transmit and receive) data with the processing circuitry 601 of the controller component 101.

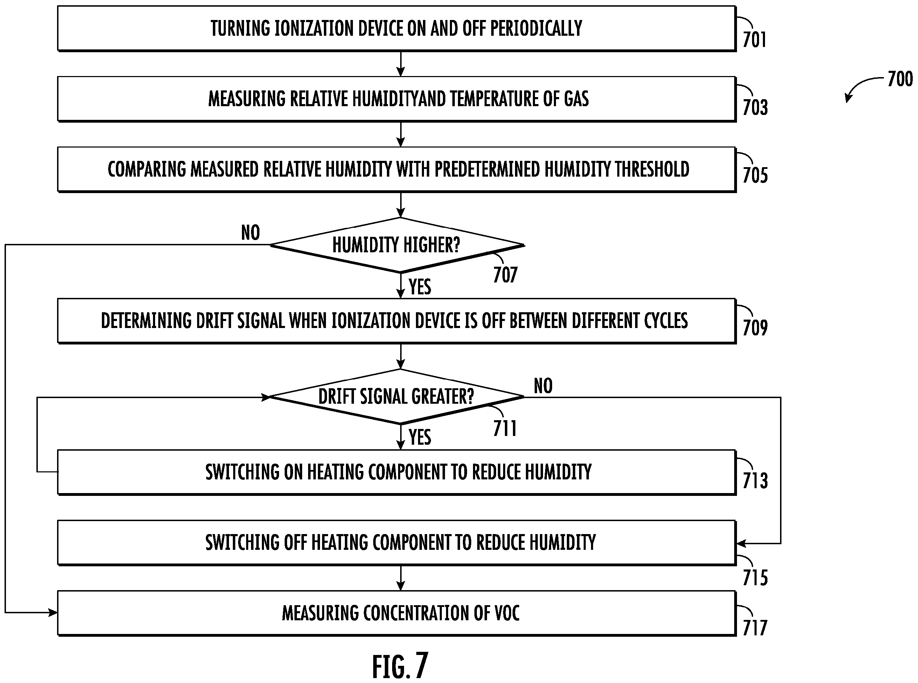

The processing circuitry 601 may be implemented as, for example, various devices including one or a plurality of microprocessors with accompanying digital signal processors; one or a plurality of processors without accompanying digital signal processors; one or a plurality of coprocessors; one or a plurality of multi-core processors; one or a plurality of controllers; processing circuits; one or a plurality of computers; and various other processing elements (including integrated circuits, such as ASICs or FPGAs, or a certain combination thereof). In some embodiments, the processing circuitry 601 may include one or more processors. In one exemplary embodiment, the processing circuitry 601 is configured to execute instructions stored in the memory 607 or otherwise accessible by the processing circuitry 601. When executed by the processing circuitry 601, these instructions may enable the controller component 101 to execute one or a plurality of the functions as described herein. No matter whether it is configured by hardware, firmware/software methods, or a combination thereof, the processing circuitry 601 may include entities capable of executing operations according to the embodiments of the present invention when correspondingly configured. Therefore, for example, when the processing circuitry 601 is implemented as an ASIC, an FPGA, or the like, the processing circuitry 601 may include specially configured hardware for implementing one or a plurality of operations described herein. Alternatively, as another example, when the processing circuitry 601 is implemented as an actuator of instructions (such as those that may be stored in the memory 607), the instructions may specifically configure the processing circuitry 601 to execute one or a plurality of algorithms and operations described herein, such as those discussed with reference to FIG. 7, FIG. 8, and FIG. 9.

The memory 607 may include, for example, a volatile memory, a non-volatile memory, or a certain combination thereof. Although illustrated as a single memory in FIG. 6, the memory 607 may include a plurality of memory components. In various embodiments, the memory 607 may include, for example, a hard disk drive, a random access memory, a cache memory, a flash memory, a Compact Disc Read-Only Memory (CD-ROM), a Digital Versatile Disk Read-Only Memory (DVD-ROM), an optical disk, a circuit configured to store information, or a certain combination thereof. The memory 607 may be configured to store information, data, application programs, instructions, and etc., so that the controller component 101 can execute various functions according to the embodiments of the present disclosure. For example, in at least some embodiments, the memory 607 is configured to cache input data for processing by the processing circuitry 601. Additionally or alternatively, in at least some embodiments, the memory 607 is configured to store program instructions for execution by the processing circuitry 601. The memory 607 may store information in the form of static and/or dynamic information. When the functions are executed, the stored information may be stored and/or used by the controller component 101.

The communication module 603 may be implemented as any apparatus included in a circuit, hardware, a computer program product or a combination thereof, which is configured to receive and/or transmit data from/to another component or apparatus. The computer program product includes computer-readable program instructions stored on a computer-readable medium (for example, the memory 607) and executed by a controller component 101 (for example, the processing circuitry 601). In some embodiments, the communication module 603 (as with other components discussed herein) may be at least partially implemented as the processing circuitry 601 or otherwise controlled by the processing circuitry 601. In this regard, the communication module 603 may communicate with the processing circuitry 601, for example, through a bus. The communication module 603 may include, for example, antennas, transmitters, receivers, transceivers, network interface cards and/or supporting hardware and/or firmware/software, and is used for establishing communication with another apparatus. The communication module 603 may be configured to receive and/or transmit any data that may be stored by the memory 607 by using any protocol that can be used for communication between apparatuses. The communication module 603 may additionally or alternatively communicate with the memory 607, the input/output module 605 and/or any other component of the controller component 101, for example, through a bus.

In some embodiments, the controller component 101 may include an input/output module 605. The input/output module 605 may communicate with the processing circuitry 601 to receive instructions input by the user and/or to provide audible, visual, mechanical or other outputs to the user. Therefore, the input/output module 605 may be in electronic communication with supporting devices, such as a keyboard, a mouse, a display, a touch screen display, and/or other input/output mechanisms. Alternatively, at least some aspects of the input/output module 605 may be implemented on a device used by the user to communicate with the controller component 101. The input/output module 605 may communicate with the memory 607, the communication module 603 and/or any other component, for example, through a bus. One or a plurality of input/output modules and/or other components may be included in the controller component 101.

Referring now to FIG. 7, a flowchart diagram illustrating an example method 700, in accordance with various embodiments of the present disclosure is provided.

As depicted in FIG. 7, in some examples, the method 700 may be performed by a processing circuitry (for example, but not limited to, an application-specific integrated circuit (ASIC), a central processing unit (CPU)). In some examples, the processing circuitry may be electrically coupled to and/or in electronic communication with other circuitries of the example apparatus, such as, but not limited to, an ionization device 102, signal electrodes 105 (105a and 105b), a heating component 107, a humidity and temperature sensor 108, a memory (such as, for example, random access memory (RAM) for storing computer program instructions), and/or a display circuitry (for rendering information on a display).

In some examples, one or more of the procedures described in FIG. 7 may be embodied by computer program instructions, which may be stored by a memory (such as a non-transitory memory) of a system employing an embodiment of the present disclosure and executed by a processing circuitry (such as a processor) of the system. These computer program instructions may direct the system to function in a particular manner, such that the instructions stored in the memory circuitry produce an article of manufacture, the execution of which implements the function specified in the flow diagram step/operation(s). Further, the system may include one or more other circuitries. Various circuitries of the system may be electronically coupled between and/or among each other to transmit and/or receive energy, data and/or information.

In some examples, embodiments may take the form of a computer program product on a non-transitory computer-readable storage medium storing computer-readable program instruction (e.g., computer software). Any suitable computer-readable storage medium may be utilized, including non-transitory hard disks, CD-ROMs, flash memory, optical storage devices, or magnetic storage devices.

At step/operation 701 of the example method 700, a processing circuitry (such as, but not limited to, the processing circuitry 601 of the controller component 101 illustrated in connection with FIG. 6, discussed above) turns the ionization device 102 on and off periodically to create a plurality of ionized gas molecules by exposing the gas to the ionization device. For example, the processing circuitry 601 of the controller component 101 may instruct the power source 103a to apply an AC voltage between the driving electrodes 103b and 103c, such that the ionization device 102 may be turned on and off periodically to create the plurality of ionized gas molecules by exposing the gas to a UV light.

In some examples, the processing circuitry 601 of the controller component 101 may instruct the power source 103a to provide an output signal 202 to the UV lamp 104. When the output signal 202 is low, the UV lamp is off, and when the output signal 202 is high, the UV lamp is on. Referring back in the top portion of FIG. 2, the UV lamp is configured to be turned on and turned off periodically when the PID 100 is enabled to detect and or measure the VOC.

At step/operation 703 of the example method 700, the processing circuitry (such as, but not limited to, the processing circuitry 601 of the controller component 101 illustrated in connection with FIG. 3, discussed above) measures and/or monitors a relative humidity and a temperature of the gas. For example, the processing circuitry 601 of the controller component 101 may instruct a humidity and temperature sensor 108 to measure and/or monitor a relative humidity and a temperature of the gas.

In some examples, the humidity and temperature sensor 108 may measure and/or monitor the relative humidity and the temperature of the gas in real time. In some examples, the processing circuitry 601 of the controller component 101 may communicate with the humidity and temperature sensor 108 and record the relative humidity and the temperature of the gas in real time.

At step/operation 705 of the example method 700, the processing circuitry (such as, but not limited to, the processing circuitry 601 of the controller component 101 illustrated in connection with FIG. 6, discussed above) compares the relative humidity with a predetermined humidity threshold.

At step/operation 707 of the example method 700, the processing circuitry (such as, but not limited to, the processing circuitry 601 of the controller component 101 illustrated in connection with FIG. 6, discussed above) compares the relative humidity with a predetermined humidity threshold. If the processing circuitry 601 of the controller component 101 determines that the relative humidity is higher than the predetermined humidity threshold, the example method may proceed to step/operation 709. If the processing circuitry 601 of the controller component 101 determines that the relative humidity is not higher than the predetermined humidity threshold, the example method may proceed to step/operation 717.

At step/operation 709 of the example method 700, the processing circuitry (such as, but not limited to, the processing circuitry 601 of the controller component 101 illustrated in connection with FIG. 6, discussed above) determines a drift signal when the ionization device is off between different cycles.

At step/operation 717 of the example method 700, the processing circuitry (such as, but not limited to, the processing circuitry 601 of the controller component 101 illustrated in connection with FIG. 6, discussed above) measures a concentration of the VOC of the gas. In some examples, the controller component 101 may determine the concentration of the VOC in the gas based on a valid signal $\Delta$Sig.

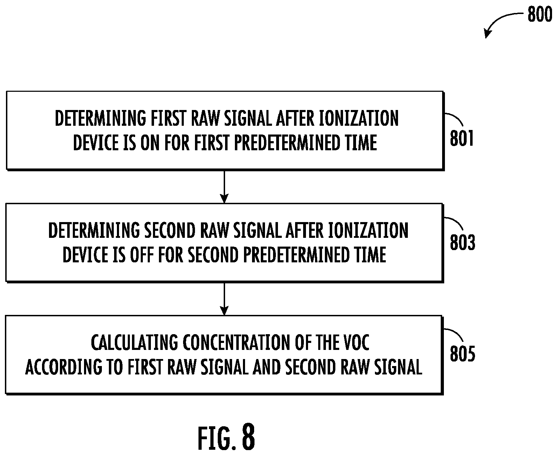

Referring now to FIG. 8, a flowchart diagram illustrating an example method 800 of performing step/operation 717 to measure the concentration of the VOC of the gas, in accordance with various embodiments of the present disclosure is provided.

At step/operation 801 of the example method 800, a processing circuitry (such as, but not limited to, the processing circuitry 601 of the controller component 101 illustrated in connection with FIG. 6, discussed above) determines a first raw signal after the ionization device is on for a first predetermined time.

In some examples, referring back to the top portion of FIG. 2, in an example first cycle, the UV lamp starts to turn on at time $T_0$. The raw signal 203 may be recorded as the first raw signal $Sig_{on}$ at time $T_1$. For example, the first predetermined time is equal to $T_1$-$T_0$.

At step/operation 803 of the example method 800, the processing circuitry (such as, but not limited to, the processing circuitry 601 of the controller component 101 illustrated in connection with FIG. 3, discussed above) determines a second raw signal after the ionization device is off for a second predetermined time.

In some examples, referring back to the top portion of FIG. 2, in the example first cycle, the UV lamp starts to turn off at time $T_2$. The raw signal 203 may be recorded as the second raw signal $Sig_{off}$ at time $T_3$. For example, the second predetermined time is equal to $T_3$-$T_2$.

At step/operation 805 of the example method 800, the processing circuitry (such as, but not limited to, the processing circuitry 601 of the controller component 101 illustrated in connection with FIG. 6, discussed above) calculates the concentration of the VOC according to the first raw signal and the second raw signal.

In some examples, the concentration of VOC in the gas may be determined based on the valid signal $\Delta Sig$, where $\Delta Sig = Sig_{on}$ - $Sig_{off}$. For example, the VOC may be proportional to the valid signal $\Delta Sig$.

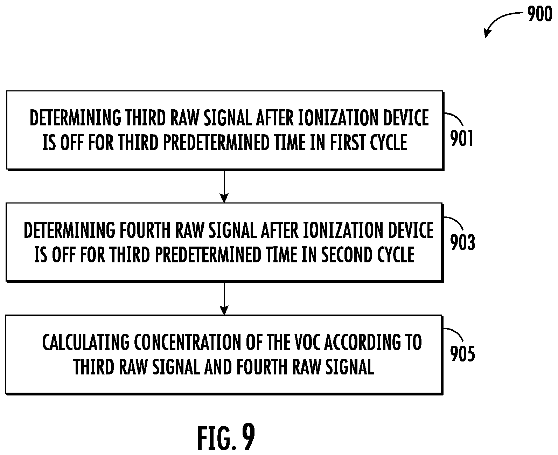

Referring now to FIG. 9, a flowchart diagram illustrating an example method 900 of performing step/operation 709 to determine a drift signal when the ionization device is off between different cycles, in accordance with various embodiments of the present disclosure is provided.

At step/operation 901 of the example method 900, a processing circuitry (such as, but not limited to, the processing circuitry 601 of the controller component 101 illustrated in connection with FIG. 6, discussed above) determines a third raw signal after the ionization device is off for a third predetermined time after the ionization device is off in a first cycle.

In some examples, referring back to the bottom portion of FIG. 2, in an example first cycle, the ultra-violet (UV) lamp starts to turn off at time $T_2$ of the example first cycle, and the raw signal 203 may be recorded as the third raw signal $Sig_{off1}$ at time $T_3$ of the example first cycle. For example, the third predetermined time may be equal to $T_3$-$T_2$.

At step/operation 903 of the example method 900, the processing circuitry (such as, but not limited to, the processing circuitry 601 of the controller component 101 illustrated in connection with FIG. 3, discussed above) determines a fourth raw signal after the ionization device is off for the third predetermined time after the ionization device is off in a second cycle.

In some examples, referring back to the bottom portion of FIG. 2, in an example second cycle, the ultra-violet (UV) lamp starts to turn off at time $T_2$ of the example second cycle, and the raw signal 203 may be recorded as the fourth raw signal $Sig_{off1}$ at time $T_3$ of the example second cycle.

At step/operation 905 of the example method 900, the processing circuitry (such as, but not limited to, the processing circuitry 601 of the controller component 101 illustrated in connection with FIG. 6, discussed above) calculates the drift signal according to the third raw signal and the fourth raw signal.

In some examples, the drift signal $\Delta Sig_{off}$ may be determined based on the following equation: $\Delta Sig_{off} = Sig_{off1}$ - $Sig_{off2}$.

Referring back to FIG. 7, at step/operation 711 of the example method 700, the processing circuitry (such as, but not limited to, the processing circuitry 601 of the controller component 101 illustrated in connection with FIG. 6, discussed above) compares the drift signal with a predetermined drift threshold. If the processing circuitry 601 of the controller component 101 determines that the drift signal is greater than the predetermined drift threshold, the example method may proceed to step/operation 713. If the processing circuitry 601 of the controller component 101 determines that the drift signal is not greater than the predetermined drift threshold, the example method may proceed to step/operation 715.

At step/operation 713 of the example method 700, the processing circuitry (such as, but not limited to, the processing circuitry 601 of the controller component 101 illustrated in connection with FIG. 6, discussed above) switches on a heating component to reduce the relative humidity.

At step/operation 715 of the example method 700, the processing circuitry (such as, but not limited to, the processing circuitry 601 of the controller component 101 illustrated in connection with FIG. 6, discussed above) switches on a heating component to reduce the humidity.

In some examples, the relative humidity of the gas will decrease when the heating component is turned on. In some examples, the method 700 may proceed to step/operation 717 when the relative humidity is decreased to be lower than the predetermined humidity threshold.

Many modifications and other embodiments of the present disclosure set forth herein will come to mind to one skilled in the art to which these embodiments pertain having the benefit of the teachings presented in the foregoing descriptions and the associated drawings. Therefore, it is to be understood that the disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

**Claims**

1. A method for measuring a volatile organic compound (VOC) by a controller component of a photo ionization detector (PID), comprising:

   measuring a relative humidity and a temperature of a gas that is input into the PID;
   comparing the measured relative humidity with a predetermined humidity threshold;
   in an instance in which the relative humidity is higher than the predetermined humidity threshold:

   determining a drift signal when an ionization device is off between different cycles;
   comparing the drift signal with a predetermined drift threshold;
   in an instance in which the drift signal is greater than the predetermined drift threshold, switching on a heating component to reduce the relative humidity; and
   in an instance in which the drift signal is smaller than the predetermined drift threshold, switching off the heating component; and

   measuring a concentration of the VOC of the gas.

2. The method according to claim 1, further comprising:
   turning the ionization device on and off periodically to create a plurality of ionized gas molecules by exposing the gas to the ionization device, wherein the plurality of ionized gas molecules are detected by signal electrodes, and the signal electrodes are electrically coupled to the controller component of the PID.

3. The method according to claim 2, wherein the controller component is configured to provide a direct current (DC) potential to the signal electrodes, such that the VOC is separated and detected.

4. The method according to claim 1, wherein the predetermined humidity threshold is determined based on a temperature of the gas.

5. The method according to claim 1, wherein measuring the concentration of the VOC in the gas comprises:

   determining a first raw signal after the ionization device is on for a first predetermined time;
   determining a second raw signal after the ionization device is off for a second predetermined time; and
   calculating the concentration of the VOC according to the first raw signal and the second raw signal.

6. The method according to claim 1, wherein determining the drift signal when the ionization device is off between different cycles comprises:

   determining a third raw signal after the ionization device is off for a third predetermined time in a first cycle;
   determining a fourth raw signal after the ionization device is off for the third predetermined time in a second cycle; and
   calculating the concentration of the VOC according to the third raw signal and the fourth raw signal.

7. The method according to claim 1, wherein the heating component is located in a detecting region of the gas.

8. The method according to claim 7, wherein the ionization device is an ultraviolet lamp projecting into the detecting region of the gas, and gas molecules with an ionization potential lower than ionization energy of a ultra-violet (UV) light are ionized.

9. The method according to claim 8, wherein a duty cycle of the ultraviolet lamp is in a range from 10% to 70%.

10. The method according to claim 7, wherein the relative humidity and temperature of the gas are measured by a humidity and temperature sensor located in the detecting region of the gas.

11. A photo ionization detector (PID) for measuring a volatile organic compound (VOC), comprising:

a controller component;
signal electrodes;
a heating component;
an ionization device, wherein the controller component is configured to:

measure a relative humidity and a temperature of a gas that is input into the PID;
compare the measured relative humidity with a predetermined humidity threshold;
in an instance in which the relative humidity is higher than the predetermined humidity threshold:

determine a drift signal of an ionization device;
compare the drift signal with a predetermined drift threshold;
in an instance in which the drift signal is greater than the predetermined drift threshold, switch on a heating component to reduce the relative humidity; and
in an instance in which the drift signal is smaller than the predetermined drift threshold, switch off the heating component.

12. The PID according to claim 11, wherein:

the controller component is further configured to turn the ionization device on and off periodically to create a plurality of ionized gas molecules by exposing the gas to the ionization device, and
the plurality of ionized gas molecules are detected by signal electrodes, and the signal electrodes are electrically coupled to the controller component.

13. The PID according to claim 12, wherein the controller component is further configured to provide a direct current (DC) potential to the signal electrodes, such that the VOC is separated and detected.

14. The PID according to claim 11, wherein the predetermined humidity threshold is determined based on a temperature of the gas.

15. The PID according to claim 11, wherein to measure the concentration of the VOC in the gas, the controller component is configure to:

determine a first raw signal after the ionization device is on for a first predetermined time;
determine a second raw signal after the ionization device is off for a second predetermined time; and
calculate the concentration of the VOC according to the first raw signal and the second raw signal.

FIG. 1

EP 4 431 932 A1

200

201 RELATIVE HUMIDITY $Th_{RH\%}$

202 UV LAMP — ON / OFF / ON / OFF / ON / OFF

203 RAW SIGNAL — $T_0$ $T_1$ $T_2$ $T_3$ / $\Delta SIG$

204 HEATING

201' RELATIVE HUMIDITY $Th_{RH\%}$

202' UV LAMP — ON / OFF / ON / OFF / ON / OFF

203' RAW SIGNAL — $\Delta SIG_{OFF}$

204' HEATING

FIG. 2

FIG. 3

400

UV LAMP OFF

UV LAMP ON

UV LIGHT INTENSITY

401

$T_{OFF}$

$T_{ON}$

$T_0$

$SIG_{ON}$

$SIG_{OFF}$

SIGNAL INTENSITY

402

FIG. 4

500

501

SIGNAL INTENSITY

$SIG_{ON1}$

$SIG_{OFF1}$

$SIG_1$

IN A CERTAIN ENVIRONMENT WITH
VOC CONCENTRATION $C_1$

$T_{OFF}$

TIME

502

SIGNAL INTENSITY

$SIG_{ON2}$

$SIG_{OFF2}$

$SIG_2$

$T_0$

$T_{ON}$

IN A CERTAIN ENVIRONMENT WITH
VOC CONCENTRATION $C_2$

$T_{OFF}$

TIME

$T_0$

$T_{ON}$

FIG. 5

FIG. 6

EP 4 431 932 A1

```
┌─────────────────────────────────────────────────────────────────────┐
│            TURNING IONIZATION DEVICE ON AND OFF PERIODICALLY          │ 701
└─────────────────────────────────────────────────────────────────────┘
                                    │
                                    ▼                                      700
┌─────────────────────────────────────────────────────────────────────┐
│              MEASURING RELATIVE HUMIDITY AND TEMPERATURE OF GAS        │ 703
└─────────────────────────────────────────────────────────────────────┘
                                    │
                                    ▼
┌─────────────────────────────────────────────────────────────────────┐
│  COMPARING MEASURED RELATIVE HUMIDITY WITH PREDETERMINED HUMIDITY     │ 705
│                           THRESHOLD                                    │
└─────────────────────────────────────────────────────────────────────┘
                                    │
              NO              ◇ HUMIDITY HIGHER? ◇                         707
                                    │ YES
                                    ▼
┌─────────────────────────────────────────────────────────────────────┐
│  DETERMINING DRIFT SIGNAL WHEN IONIZATION DEVICE IS OFF BETWEEN        │ 709
│                       DIFFERENT CYCLES                                 │
└─────────────────────────────────────────────────────────────────────┘
                                    │
                          ◇ DRIFT SIGNAL GREATER? ◇        NO              711
                                    │ YES
                                    ▼
┌─────────────────────────────────────────────────────────────────────┐
│         SWITCHING ON HEATING COMPONENT TO REDUCE HUMIDITY             │ 713
└─────────────────────────────────────────────────────────────────────┘
                                    │
                                    ▼
┌─────────────────────────────────────────────────────────────────────┐
│         SWITCHING OFF HEATING COMPONENT TO REDUCE HUMIDITY            │ 715
└─────────────────────────────────────────────────────────────────────┘
                                    │
                                    ▼
┌─────────────────────────────────────────────────────────────────────┐
│                   MEASURING CONCENTRATION OF VOC                       │ 717
└─────────────────────────────────────────────────────────────────────┘
```

FIG. 7

800

801 — DETERMINING FIRST RAW SIGNAL AFTER IONIZATION DEVICE IS ON FOR FIRST PREDETERMINED TIME

803 — DETERMINING SECOND RAW SIGNAL AFTER IONIZATION DEVICE IS OFF FOR SECOND PREDETERMINED TIME

805 — CALCULATING CONCENTRATION OF THE VOC ACCORDING TO FIRST RAW SIGNAL AND SECOND RAW SIGNAL

FIG. 8

900

```
┌─────────────────────────────────────────────────┐
│ DETERMINING THIRD RAW SIGNAL AFTER IONIZATION    │
│ DEVICE IS OFF FOR THIRD PREDETERMINED TIME IN    │──901
│ FIRST CYCLE                                       │
└─────────────────────────────────────────────────┘
                        │
                        ▼
┌─────────────────────────────────────────────────┐
│ DETERMINING FOURTH RAW SIGNAL AFTER IONIZATION   │
│ DEVICE IS OFF FOR THIRD PREDETERMINED TIME IN    │──903
│ SECOND CYCLE                                      │
└─────────────────────────────────────────────────┘
                        │
                        ▼
┌─────────────────────────────────────────────────┐
│ CALCULATING CONCENTRATION OF THE VOC ACCORDING   │
│ TO THIRD RAW SIGNAL AND FOURTH RAW SIGNAL        │──905
└─────────────────────────────────────────────────┘
```

FIG. 9

Europäisches
Patentamt

European
Patent Office

Office européen
des brevets

# EUROPEAN SEARCH REPORT

**Application Number**

EP 24 15 8479

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 770 595 A1 (HONEYWELL INT INC [US]) 27 January 2021 (2021-01-27) * abstract * * figures 1-8 * * paragraph [0005] - paragraph [0013] * * paragraph [0015] - paragraph [0058] * ----- | 1,3,8, 11,13 | INV. G01N27/624 G01N33/00 ADD. G01N1/22 G01N1/44 |
| A | WO 2009/092007 A1 (GEN DYNAMICS ARMAMENT & TECH [US]) 23 July 2009 (2009-07-23) * abstract * * figures 1-6 * * paragraph [0009] - paragraph [0011] * * paragraph [0025] - paragraph [0076] * ----- | 1,3,8, 11,13 | |
| A | US 2012/136268 A1 (LI BO [US] ET AL) 31 May 2012 (2012-05-31) * the whole document * ----- | 1-15 | |

**TECHNICAL FIELDS SEARCHED (IPC)**

G01N

The present search report has been drawn up for all claims

| Place of search | Date of completion of the search | Examiner |
|---|---|---|
| The Hague | 14 June 2024 | De Kroon, Arnoud |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 8479

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2024

| Patent document cited in search report | | Publication date | Patent family member(s) | | Publication date |
|---|---|---|---|---|---|
| EP 3770595 | A1 | 27-01-2021 | CN | 112179975 A | 05-01-2021 |
| | | | EP | 3770595 A1 | 27-01-2021 |
| | | | EP | 4215910 A1 | 26-07-2023 |
| | | | US | 2021003542 A1 | 07-01-2021 |
| | | | US | 2023408472 A1 | 21-12-2023 |
| WO 2009092007 | A1 | 23-07-2009 | US | 2012068061 A1 | 22-03-2012 |
| | | | WO | 2009092007 A1 | 23-07-2009 |
| US 2012136268 | A1 | 31-05-2012 | CN | 102565183 A | 11-07-2012 |
| | | | EP | 2458375 A1 | 30-05-2012 |
| | | | JP | 5923284 B2 | 24-05-2016 |
| | | | JP | 2012118063 A | 21-06-2012 |
| | | | US | 2012136268 A1 | 31-05-2012 |

EPO FORM P0459

For more details about this annex : see Official Journal of the European Patent Office, No. 12/82